# FASCICULE DE BREVET EUROPEEN

(11) **EP 4 638 398 B1**
(45) Date de publication et mention de la délivrance du brevet: **02.12.2026**
(21) Numéro de dépôt: 23824919.7
(22) Date de dépôt: 12.12.2023
(51) Int. Cl.: C07C 1/207, C07C 45/29, C07C 51/15, C07C 11/167, C07C 47/06, C07C 55/14, C12F 3/02, C12P 7/10, C12P 19/02, C12P 19/14

(54) **CONVERSION D'UNE CHARGE HYDROCARBONEE ISSUE DE LA BIOMASSE POUR PRODUIRE DE L'ACIDE ADIPIQUE**
UMWANDLUNG EINES KOHLENWASSERSTOFFEINSATZES AUS BIOMASSE FÜR DIE HERSTELLUNG VON ADIPINSÄURE
CONVERSION OF A HYDROCARBON CHARGE FROM BIOMASS FOR PRODUCING ADIPIC ACID

(30) Priorité: 20.12.2022 FR 2213916
(43) Date de publication de la demande: 29.10.2025
(73) Titulaire: IFP Energies nouvelles, 92500 Rueil-Malmaison (FR)
(72) Inventeur: LAROCHE, Catherine, 92852 RUEIL-MALMAISON CEDEX (FR); CHAHEN, Ludovic, 92852 RUEIL-MALMAISON CEDEX (FR)
(74) Mandataire: IFP Energies nouvelles
(86) Numéro de dépôt international: PCT/EP2023/085336
(87) Numéro de publication internationale: WO 2024/132695

(56) Documents cités:
- EP-A1- 3 995 474
- WO-A1-2018/202724
- US-A1- 2016 273 011

## Description

### Domaine technique

L'invention concerne un procédé de traitement de biomasse, de préférence de biomasse lignocellulosique pour produire de l'acide adipique à partir d'une seule source de carbone biosourcée tout en maximisant le rendement carbone.

Le procédé de fermentation de la biomasse lignocellulosique permet de produire de l'éthanol et du dioxyde de carbone (CO₂) qui peuvent être convertis avantageusement, par rapport à l'art antérieur, en acide adipique.

### Etat de la technique

L'acide adipique est un composé chimique utilisé comme matière première en tant que composé intermédiaire notamment pour la synthèse de polyamides, de polyesters ou encore de polyuréthannes.

A ce jour, plusieurs procédés de fabrication de l'acide adipique sont connus et exploités industriellement. Le procédé principal exploité industriellement consiste à oxyder par l'oxygène le cyclohexane pour obtenir des composés intermédiaires cyclohexanol et cyclohexanone, généralement un mélange de ces deux composés. Dans une étape ultérieure, les composés intermédiaires, cyclohexanol/cyclohexanone, sont oxydés en acide adipique par de l'acide nitrique, en présence d'un catalyseur.

Le brevet US5487987 décrit une voie de synthèse de l'acide adipique à partir de la biomasse pour former dans un premier temps du catéchol qui est ensuite transformé en acide cis,cis-muconique qui peut subir une réduction pour former l'acide adipique.

La demande de brevet US2011/0172475 décrit quant à elle une voie de synthèse d'acide adipique en passant par la production d'isobutanol à partir de la biomasse. L'isobutanol est transformé par la suite en 1,3-butadiène qui est lui-même transformé en adiponitrile puis enfin en acide adipique.

Le 1,3-butadiène est un produit stratégiquement intéressant pour la synthèse d'acide adipique. Ce composé peut être obtenu par la déshydratation de bioéthanol. Ce type de procédé est divulgué notamment dans les demandes WO21193457, WO21006252 ou encore dans la demande WO21052968. Il est bien connu que le bioéthanol peut être produit par fermentation de sucres provenant de diverses biomasses. Toutefois, les fermentations pour produire de l'éthanol n'ont généralement pas de bons rendements « carbone » car une partie de celui-ci est perdu sous la forme de CO₂. De plus, l'éthanol produit à partir de biomasse dite de 1ère génération (1G) est en concurrence avec l'agroalimentaire et perdre une grande partie de cette ressource sous la forme d'un gaz à effet de serre est encore plus problématique. La demande WO2018/202724 décrit la synthèse d'acide adipique à partir du 1,3-butadiène.

L'invention a pour but de remédier à l'ensemble des inconvénients cités ci-avant. Plus précisément, l'invention a pour but de mettre au point un procédé de traitement de la biomasse lignocellulosique dite de seconde génération (2G) pour produire de l'acide adipique à partir d'éthanol et de CO₂ issu de la fermentation.

L'invention a pour but de remédier à l'ensemble des inconvénients cités ci-avant. Plus précisément, l'invention a pour but de mettre au point un procédé de traitement de la biomasse, de préférence une biomasse lignocellulosique dite de seconde génération (2G), pour produire de l'acide adipique à partir d'éthanol et de CO₂ issus de la fermentation.

### Objets de l'invention

Dans le contexte précédemment décrit, un premier objet de la présente description est de surmonter les problèmes de l'art antérieur et de valoriser le carbone, et en particulier le carbone biosourcé sous forme CO₂ en composés à haute valeur ajoutée, et en particulier en acide adipique. Spécifiquement, la présente invention concerne un procédé de production de d'acide adipique selon un arrangement d'étapes, permettant de convertir la biomasse en éthanol et en CO₂ puis de convertir ces produits en acide adipique, en utilisant une ou plusieurs des étapes suivantes en plus ou en remplacement de certaines étapes des synthèses classiques de l'acide adipique.

Selon un premier aspect, la présente invention concerne un procédé de conversion de biomasse, de préférence une biomasse lignocellulosique, en acide adipique comprenant successivement :
a) une étape de traitement de la biomasse pour produire de l'éthanol et du CO₂ ;
b) une étape de conversion de l'éthanol obtenu à l'issue de l'étape a) pour obtenir du 1,3-butadiène ;
c) une étape de synthèse d'acide adipique à partir du 1,3-butadiène obtenu à l'issue de l'étape
b) et du CO₂ obtenu à l'issue de l'étape a).

L'invention repose sur la valorisation du CO₂ qui est un sous-produit formé pendant l'étape de fermentation de la biomasse en éthanol et éventuellement de la réutilisation de l'eau produite pendant l'étape de déshydratation dans les autres étapes du procédé selon l'invention. Cette invention présente donc un enchainement d'opérations unitaires servant à maximiser le rendement carbone de la synthèse d'acide adipique à partir de biomasse, de préférence une biomasse lignocellulosique, et encore plus préférentiellement une biomasse lignocellulosique dite de seconde génération (2G).

Selon un ou plusieurs modes de réalisation, l'étape a) comprend les sous-étapes suivantes :
a1) une étape de prétraitement de la biomasse pour obtenir un substrat prétraité ;
a2) une étape d'hydrolyse enzymatique ou chimique du substrat prétraité obtenu à l'issue de l'étape a1) pour obtenir un moût d'hydrolyse enzymatique ou chimique ;
a3) une étape de fermentation alcoolique du moût d'hydrolyse enzymatique ou chimique obtenu à l'issue de l'étape a2) pour obtenir de l'éthanol et du CO₂.

Selon un ou plusieurs modes de réalisation, la sous-étape a1) est réalisée par explosion à la vapeur en condition acide à une température comprise entre 150°C et 250°C et pendant une durée comprise entre 5 minutes et 30 minutes.

Selon un ou plusieurs modes de réalisation, la sous-étape a2) est réalisée par hydrolyse enzymatique en présence des cellulases de *Trichoderma reesei.*

Selon un ou plusieurs modes de réalisation, l'étape b) comprend les sous-étapes suivantes :
b1) une étape de conversion de l'éthanol en acétaldéhyde comprenant au moins une section réactionnelle alimentée au moins par une fraction de l'effluent riche en éthanol issu de l'étape b5), opérée à une pression comprise entre 0,1 MPa et 1,0 MPa et à une température comprise entre 200°C et 500°C en présence d'un catalyseur, et une section de séparation permettant de séparer l'effluent de ladite section réactionnelle en au moins un effluent hydrogène sous forme gazeuse et un effluent éthanol/acétaldéhyde sous forme liquide ;
b2) une étape de conversion en butadiène comprenant au moins une section réactionnelle alimentée au moins par une fraction dudit effluent éthanol/acétaldéhyde issu de l'étape b1), par un effluent liquide riche en éthanol issu de l'étape b3), par une fraction de l'effluent riche en acétaldéhyde issu de l'étape b5), opérée en présence d'un catalyseur, à une température comprise entre 300°C et 400°C, et à une pression comprise entre 0,1 MPa et 1,0 MPa, les débits d'alimentation étant réglés de telle sorte que le rapport molaire éthanol/acétaldéhyde en entrée de ladite section réactionnelle est compris entre 1 et 5, et une section de séparation permettant de séparer l'effluent de ladite section réactionnelle en au moins un effluent gazeux et un effluent liquide ;
b3) une étape de traitement de l'hydrogène comprenant au moins une section de compression comprimant ledit effluent hydrogène issu de l'étape b1) à une pression comprise entre 0,1 MPa et 1,0 MPa, et une section de lavage gaz-liquide alimentée à une température comprise entre 15°C et -30°C par une fraction dudit effluent éthanol issu de l'étape b5) et par une fraction dudit effluent éthanol/acétaldéhyde issu de l'étape b1), et alimentée à une température comprise entre 25°C et 60°C par ledit effluent hydrogène comprimé, et produisant au moins un effluent liquide riche en éthanol et un effluent hydrogène purifié ;
b4) une étape d'extraction du butadiène comprenant au moins une section de compression comprimant ledit effluent gazeux issu de l'étape b2) à une pression comprise entre 0,1 MPa et 1,0 MPa, une section de lavage gaz-liquide comprenant une colonne de lavage alimentée en tête à une température comprise entre 20°C et -20°C par un flux éthanol constitué de ladite charge éthanol du procédé et/ou d'une fraction de l'effluent éthanol issu de l'étape b5) et en fond par ledit effluent gazeux issue de l'étape b2) et refroidi, et une section de distillation opérée à une pression comprise entre 0,1 MPa et 1 MPa, alimentée au moins par l'effluent liquide issu de ladite étape b2) et par l'effluent liquide de ladite section de lavage gaz-liquide, ladite étape b4) produisant au moins un effluent sous-produits gazeux, un effluent butadiène brut, et un effluent éthanol / acétaldéhyde / eau;
b4') une étape de première purification du butadiène comprenant au moins une section de lavage gaz-liquide alimentée en fond par l'effluent butadiène brut issu de b4) et en tête par un flux d'eau pouvant être un flux d'eau d'origine externe audit procédé de production de butadiène et/ou une fraction de l'effluent eau issu de l'étape b5), ladite section de lavage produisant en tête un effluent butadiène pré-purifié et en fond un effluent eau usée ;
b4") une étape ultérieure de purification du butadiène, alimentée au moins par ledit effluent butadiène pré-purifié issu de ladite étape b4'), et produisant au moins un effluent butadiène purifié ;
b5) une étape traitement des effluents alimentée au moins par le raffinat eau / éthanol / acétaldéhyde issu de l'étape b5'), et produisant au moins un effluent riche en éthanol, un effluent riche en acétaldéhyde et un effluent riche en eau ;
b5') une étape d'élimination des impuretés et des huiles brunes, alimentée au moins par l'effluent éthanol/acétaldéhyde/eau issu de l'étape b4), et par l'effluent riche en eau issu de l'étape b5), et produisant au moins un raffinat eau / éthanol / acétaldéhyde, un effluent huiles brunes légères et un effluent huiles brunes lourdes ;
b6) une étape de lavage à l'eau, alimentée par l'effluent sous-produits gazeux issu de l'étape b4), ainsi que par une fraction de l'effluent riche en eau issu de ladite étape b5) et produisant au moins un effluent eau alcoolisée.

Selon un ou plusieurs modes de réalisation, l'étape c) comprend les sous-étapes suivantes :
c1) une étape de couplage C-C entre deux molécules de CO₂ et une molécule de 1,3-butadiène ;
c2) une étape de séparation des acides hexènedioïques ;
c3) une étape de réduction des diacides hexènedioïques formés dans à l'étape c1) en acide adipique.

Selon un ou plusieurs modes de réalisation, la sous-étape c1) est réalisée en présence d'un précurseur métallique, un ligand et un réducteur dans un solvant, caractérisé en ce que :
- le précurseur métallique est un sel de nickel (II);
- le ligand est un ligand bidentate diazoté ;
- le réducteur est un métal choisi parmi le zinc ou le manganèse ;
- le solvant est un solvant polaire aprotique.

Selon un ou plusieurs modes de réalisation, la sous-étape c1) est réalisée à une température comprise entre 5°C et 70°C.

Selon un ou plusieurs modes de réalisation, la sous-étape c3) est réalisée en présence d'un catalyseur et d'un agent de réduction, ledit catalyseur étant le palladium sur charbon actif, et l'agent de réduction étant le l'hydrogène purifié provenant de la sous-étape b3).

Selon un ou plusieurs modes de réalisation, la biomasse est une biomasse lignocellulosique.

Selon un deuxième aspect, la présente invention concerne une installation apte à mettre le procédé selon l'invention, ladite installation comprenant :
- une première section réactionnelle permettant de produire de l'éthanol et du CO₂ à partir de la biomasse ;
- une deuxième section réactionnelle de conversion de l'éthanol en 1,3-butadiène et en dihydrogène ; et
- une troisième section réactionnelle permettant de produire l'acide adipique à partir du 1,3-butadiène, du CO₂ et de dihydrogène.

### Liste des figures

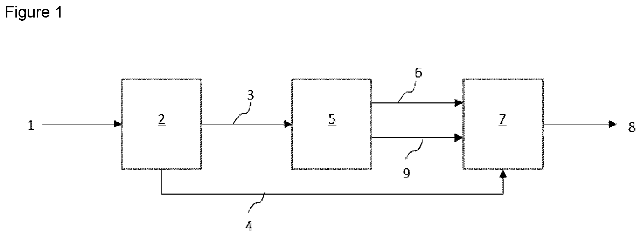

La Figure 1 montre une représentation schématique du procédé et de l'installation selon la présente invention permettant de produire de l'acide adipique à partir de la biomasse, de préférence lignocellulosique.

### Description des modes de réalisation

Des modes de réalisation du procédé selon le premier aspect de l'invention et de l'installation selon le deuxième aspect de l'invention vont maintenant être décrits en détail. Dans la description détaillée suivante, de nombreux détails spécifiques sont exposés afin de fournir une compréhension plus approfondie du procédé et de l'installation. Cependant, il apparaîtra à l'homme du métier que le procédé et l'installation peuvent être mis en oeuvre sans ces détails spécifiques. Dans d'autres cas, des caractéristiques bien connues n'ont pas été décrites en détail pour éviter de compliquer inutilement la description.

Dans la présente demande, le terme « comprendre » est synonyme de (signifie la même chose que) « inclure » et « contenir », et est inclusif ou ouvert et n'exclut pas d'autres éléments non récités. Il est entendu que le terme « comprendre » inclut le terme exclusif et fermé « consister ». En outre, dans la présente description, un effluent comprenant essentiellement ou uniquement des composés A correspond à un effluent comprenant au moins 90% en poids, préférablement au moins 95% en poids, très préférablement au moins 99% en poids, de composés A.

Dans la présente demande, les groupes d'éléments chimiques sont donnés par défaut selon la classification CAS (CRC Handbook of Chemistry and Physics, éditeur CRC press, rédacteur en chef D.R. Lide, 81ème édition, 2000-2001). Par exemple, le groupe VIII (ou VIIIB) selon la classification CAS correspond aux métaux des colonnes 8, 9 et 10 selon la nouvelle classification IUPAC ; le groupe VIB selon la classification CAS correspond aux métaux de la colonne 6 selon la nouvelle classification IUPAC.

Dans la présente demande, on entend par biomasse toute charge produite par voie biologique, de préférence par fermentation de sucres issus par exemple des cultures de plantes sucrières comme la canne à sucre (saccharose, glucose, fructose, et sucrose), des betteraves, ou encore des plantes amylacées (amidon) ou de la biomasse lignocellulosique ou de cellulose hydrolysée (glucose majoritaire et xylose, galactose), contenant des quantités variables d'eau. De préférence, la biomasse est une biomasse lignocellulosique, et encore plus préférentiellement une biomasse lignocellulosique dite de seconde génération (2G).

La présente invention peut se définir comme un procédé comprenant un enchaînement d'étapes réactionnelles permettant de produire de l'acide adipique à partir de biomasse, de préférence de biomasse lignocellulosique, et encore plus préférentiellement de biomasse lignocellulosique dite de seconde génération (2G). Plus particulièrement, la présente invention concerne un procédé de conversion de biomasse en acide adipique comprenant successivement :
a) une étape de traitement de la biomasse pour produire de l'éthanol et du CO₂ ;
b) une étape de conversion de l'éthanol obtenu à l'issue de l'étape a) pour du 1,3-butadiène ;
c) une étape de synthèse d'acide adipique à partir du 1,3-butadiène obtenu à l'issue de l'étape
b) et du CO₂ obtenu à l'issue de l'étape a).

Par ailleurs, la présente invention peut aussi se définir comme une installation apte à mettre en oeuvre le procédé selon l'invention, tel qu'illustré en figure 1, ladite installation comprenant notamment :
- une première section réactionnelle **2** permettant de produire de l'éthanol **3** et du CO₂ **4** à partir de la biomasse **1,** de préférence de la biomasse lignocellulosique ;
- une deuxième section réactionnelle **5** de conversion de l'éthanol **3** en 1,3-butadiène **6** et en hydrogène **9** ; et
- une troisième section réactionnelle **7** permettant de produire de l'acide adipique **8** à partir du 1,3-butadiène **6,** de l'hydrogène **9** et du CO₂ **4.**

### Première section réactionnelle (étape a) du procédé selon l'invention)

La première section réactionnelle **2** permet de produire de l'éthanol **3** et du CO₂ **4** à partir de la biomasse **1.**

Dans un mode de réalisation selon l'invention, la biomasse utilisée dans le procédé est une biomasse lignocellulosique, de préférence une biomasse lignocellulosique dite de seconde génération. Les bois de feuillus et les pailles de céréales sont les substrats les plus utilisés. Ils sont constitués pour la plupart d'environ 40% à 50% de cellulose, 20% à 25% d'hémicellulose et de 15% à 25% de lignine. D'autres ressources, cultures forestières dédiées, résidus de plantes alcooligènes, sucrières et céréalières, résidus de l'industrie papetière et des produits de transformation des matériaux cellulosiques et lignocellulosiques sont utilisables.

Dans un mode de réalisation selon l'invention, le procédé de transformation de la biomasse en éthanol comprend plus particulièrement les sous-étapes suivantes :
a1) une étape de prétraitement de la biomasse pour obtenir un substrat prétraité ;
a2) une étape d'hydrolyse enzymatique ou chimique du résidu cellulosique obtenu à l'issue de l'étape a1) pour obtenir un moût d'hydrolyse enzymatique ou chimique ;
a3) une étape de fermentation alcoolique du moût d'hydrolyse enzymatique ou chimique obtenu à l'issue de l'étape a2) pour obtenir de l'éthanol et du CO₂.

### Prétraitement physico-chimique (étape a1))

L'étape de prétraitement a1) permet l'obtention d'un substrat prétraité comprenant les sucres contenus dans les hémicelluloses sous forme de monomères, essentiellement des pentoses, comme le xylose et l'arabinose, et des hexoses, comme le galactose, le mannose et le glucose, et d'améliorer l'accessibilité de la cellulose engluée dans la matrice de lignine et hémicelluloses. De nombreuses technologies existent : les traitements par cuissons acides, par cuissons alcalines, par explosion à la vapeur, ou les traitements organo-solvant (ou organosolv pulping selon la terminologie anglo-saxonne). L'efficacité du prétraitement se mesure par le taux de récupération des hémicelluloses et par la susceptibilité à l'hydrolyse du résidu cellulosique. Les prétraitements acides, en condition douce, et par explosion à la vapeur sont les mieux adaptés car ils permettent une récupération totale des pentoses et une bonne accessibilité de la cellulose à l'hydrolyse.

De manière préférentielle, l'étape de prétraitement a1) est réalisée par explosion à la vapeur en condition acide à une température comprise avantageusement entre 150°C et 250°C et pendant une durée comprise avantageusement entre 5 et 30 minutes. Dans ce mode de réalisation, l'étape a1) permet de transformer les hémicelluloses en monomères tout en minimisant les pertes, en furfural notamment, le xylose étant le sucre majoritaire. Les sucres libérés sont ensuite extraits par lavage en phase aqueuse. Le résidu solide (i.e. le substrat prétraité) obtenu à l'issue de l'extraction ne contient alors que de la cellulose et de la lignine.

### Hydrolyse enzymatique ou chimique (étape a2))

Le substrat prétraité obtenu à l'issue de l'étape a1) est ensuite hydrolysé, soit par voie acide (i.e. par voie chimique), soit par voie enzymatique avec l'utilisation d'enzymes cellulolytiques et/ou hémicellulolytiques. Des microorganismes, comme les champignons appartenant aux genres *Trichoderma, Aspergillus, Penicillium* ou *Schizophyllum,* ou les bactéries anaérobies appartenant par exemple au genre *Clostridium,* produisent ces enzymes, contenant notamment les cellulases et les xylanases, adaptées à l'hydrolyse totale des polymères constituant les végétaux.

La voie acide, effectuée à l'aide d'acide fort, et plus particulièrement avec de l'acide sulfurique, est efficace mais requiert d'importantes quantités de produits chimiques (acide puis base pour la neutralisation). L'hydrolyse enzymatique ne présente pas cet inconvénient ; elle s'effectue par ailleurs dans des conditions douces et est efficace.

De manière préférentielle, le substrat prétraité, débarrassé ou non de la fraction hémicellulosique hydrolysée et le cas échéant de la lignine, est hydrolysé par les enzymes cellulolytiques et/ou hémicellulolytiques produites par les souches spécialisées, les cellulases de *Trichoderma reesei* étant les plus efficaces et les plus appropriées lorsque les substrats carbonés sont issus de la biomasse cellulosique ou lignocellulosique. Le substrat prétraité à hydrolyser est de préférence mis en suspension en phase aqueuse à raison de 6 à 25 % de matière sèche, de préférence 10 à 20 %, le pH est ajusté entre 4 et 5,5, de préférence entre 4,8 et 5,2 et la température entre 40°C et 60°C, de préférence entre 45°C et 50°C. La réaction d'hydrolyse est démarrée par ajout des cellulases ; la quantité habituellement utilisée est de 10 mg à 30 mg de protéines excrétées par gramme de substrat prétraité. La réaction dure généralement de 15 heures à 48 heures selon l'efficacité du prétraitement, la composition du mélange de cellulases et la quantité d'enzymes ajoutées. La réaction est suivie par dosage des sucres libérés, notamment le glucose. La solution sucrée (moût) est ensuite séparée de la fraction solide non hydrolysée, essentiellement constituée de lignine, par filtration ou centrifugation ; ce moût est utilisé pour la fermentation éthanolique. Lorsque la fraction cellulosique a été débarrassée des hémicelluloses hydrolysées à l'étape de traitement, le glucose est le sucre majoritaire contenu dans le moût.

### Fermentation (étape a3))

La fermentation alcoolique est un processus biochimique par lequel les sucres (glucides, principalement le glucose) contenues dans le moût sont transformés en alcool, préférentiellement en éthanol, dans un milieu liquide, privé d'air (anaérobie). L'étape de fermentation des sucres pour obtenir de l'éthanol est bien connue de l'homme de l'art.

La fermentation alcoolique s'effectue préférentiellement à une température comprise entre 25°C et 32°C. Pour une description plus complète des procédés fermentaires classiques, on peut se reporter à l'ouvrage 'Les Biocarburants, Etat des lieux, perspectives et enjeux du développement, Daniel Ballerini, Editions Technip, 2006'.

**En** général, l'éthanol est séparé du moût de fermentation par distillation et le résidu est constitué des vinasses de distillation. Une distillation régulière ou en continue de l'éthanol est nécessaire car au-delà de 14% d'éthanol, certaines levures peuvent être « empoisonnées » et cela engendre une perte de productivité. La distillation est conduite pour permettre l'obtention d'une charge éthanolique adaptée au procédé de déshydratation décrit ultérieurement.

Le CO₂ est récupéré sous forme de gaz en sortie de fermenteur. Selon un aspect essentiel de l'invention, le CO₂ obtenu à l'issue de l'étape de fermentation est envoyé au moins en partie à l'étape de synthèse de l'acide adipique (étape c) du procédé selon l'invention). Le CO₂ peut être compressé avant stockage ou avant son utilisation à l'aide de compresseurs.

Le résidu de la fermentation éthanolique, après séparation de l'éthanol, peut être utilisé comme source de carbone inductrice ou comme source de carbone principale pour la production d'enzymes. La concentration de ce résidu est de préférence ajustée pour obtenir la concentration en source de carbone la mieux adaptée au procédé de production d'enzymes cellulolytiques et/ou hémicellulolytiques.

L'étape d'hydrolyse enzymatique et de fermentation peuvent être réalisées simultanément (procédé SSF pour « *Simultaneous Saccharification and Fermentation »* selon la terminologie anglo-saxonne), puis sont avantageusement suivies d'une étape de distillation et de séparation de l'alcool obtenu.

### Deuxième section réactionnelle (étape b) du procédé selon l'invention)

La deuxième section réactionnelle **5** permet de produire du 1,3-butadiène **6** à partir de l'éthanol **3** issue de la première section réactionnelle **2.** Il existe de nombreux procédés dans la littérature permettant d'obtenir du 1,3-butadiène à partir d'éthanol, et notamment le procédé divulgué dans la demande de brevet FR3026100 qui est décrit ici à titre d'exemple.

Avantageusement, la charge d'éthanol utilisé dans l'étape b) du procédé selon l'invention est une charge comprenant au moins 80% poids d'éthanol par rapport au poids total de la charge, préférentiellement au moins 90% poids, et de manière préférée au moins 93% poids. De manière très préférée, ladite charge éthanol répond aux spécifications d'éthanol carburant EN 15376.

Selon un mode de réalisation selon l'invention, l'étape b) du procédé selon l'invention comprend les sous-étapes suivantes :
b1) une étape de conversion de l'éthanol en acétaldéhyde comprenant au moins une section réactionnelle alimentée au moins par une fraction de l'effluent riche en éthanol issu de l'étape b5), opérée à une pression comprise entre 0,1 MPa et 1,0 MPa et à une température comprise entre 200°C et 500°C en présence d'un catalyseur, et une section de séparation permettant de séparer l'effluent de ladite section réactionnelle en au moins un effluent hydrogène sous forme gazeuse et un effluent éthanol/acétaldéhyde sous forme liquide ;
b2) une étape de conversion en butadiène comprenant au moins une section réactionnelle alimentée au moins par une fraction dudit effluent éthanol/acétaldéhyde issu de l'étape b1), par un effluent liquide riche en éthanol issu de l'étape b3), par une fraction de l'effluent riche en acétaldéhyde issu de l'étape b5), opérée en présence d'un catalyseur, à une température comprise entre 300°C et 400°C, et à une pression comprise entre 0,1 MPa et 1,0 MPa, les débits d'alimentation étant réglés de telle sorte que le rapport molaire éthanol/acétaldéhyde en entrée de ladite section réactionnelle est compris entre 1 et 5, et une section de séparation permettant de séparer l'effluent de ladite section réactionnelle en au moins un effluent gazeux et un effluent liquide ;
b3) une étape de traitement de l'hydrogène comprenant au moins une section de compression comprimant ledit effluent hydrogène issu de l'étape b1) à une pression comprise entre 0,1 MPa et 1,0 MPa, et une section de lavage gaz-liquide alimentée à une température comprise entre 15°C et -30°C par une fraction dudit effluent éthanol issu de l'étape b5) et par une fraction dudit effluent éthanol/acétaldéhyde issu de l'étape b1), et alimentée à une température comprise entre 25°C et 60°C par ledit effluent hydrogène comprimé, et produisant au moins un effluent liquide riche en éthanol et un effluent hydrogène purifié ;
b4) une étape d'extraction du butadiène comprenant au moins une section de compression comprimant ledit effluent gazeux issu de l'étape b2) à une pression comprise entre 0,1 MPa et 1,0 MPa, une section de lavage gaz-liquide comprenant une colonne de lavage alimentée en tête à une température comprise entre 20°C et -20°C par un flux éthanol constitué de ladite charge éthanol du procédé et/ou d'une fraction de l'effluent éthanol issu de l'étape b5) et en fond par ledit effluent gazeux issue de l'étape b2) et refroidi, et une section de distillation opérée à une pression comprise entre 0,1 MPa et 1 MPa, alimentée au moins par l'effluent liquide issu de ladite étape b2) et par l'effluent liquide de ladite section de lavage gaz-liquide, ladite étape b4) produisant au moins un effluent sous-produits gazeux, un effluent butadiène brut, et un effluent éthanol / acétaldéhyde / eau;
b4') une étape de première purification du butadiène comprenant au moins une section de lavage gaz-liquide alimentée en fond par l'effluent butadiène brut issu de b4) et en tête par un flux d'eau pouvant être un flux d'eau d'origine externe audit procédé de production de butadiène et/ou une fraction de l'effluent eau issu de l'étape b5), ladite section de lavage produisant en tête un effluent butadiène pré-purifié et en fond un effluent eau usée ;
b4") une étape ultérieure de purification du butadiène, alimentée au moins par ledit effluent butadiène pré-purifié issu de ladite étape b4'), et produisant au moins un effluent butadiène purifié ;
b5) une étape traitement des effluents alimentée au moins par le raffinat eau / éthanol / acétaldéhyde issu de l'étape b5'), et produisant au moins un effluent riche en éthanol, un effluent riche en acétaldéhyde et un effluent riche en eau ;
b5') une étape d'élimination des impuretés et des huiles brunes, alimentée au moins par l'effluent éthanol/acétaldéhyde/eau issu de l'étape b4), et par l'effluent riche en eau issu de l'étape b5), et produisant au moins un raffinat eau / éthanol / acétaldéhyde, un effluent huiles brunes légères et un effluent huiles brunes lourdes ;
b6) une étape de lavage à l'eau, alimentée par l'effluent sous-produits gazeux issu de l'étape b4), ainsi que par une fraction de l'effluent riche en eau issu de ladite étape b5) et produisant au moins un effluent eau alcoolisée.

Les étapes b1) à b6) sont décrites en détail ci-après.

### Étape b1) de conversion de l'éthanol en acétaldéhyde

Conformément à un mode de réalisation selon l'invention, une étape b1) de conversion de l'éthanol en acétaldéhyde comprend au moins une section réactionnelle alimentée au moins par une fraction de l'effluent riche en éthanol issu de l'étape b5), ladite fraction constituant de préférence au moins 10% du débit dudit effluent riche en éthanol issu de l'étape b5), et optionnellement avantageusement alimentée par au moins une fraction de ladite charge éthanol, et une section de séparation permettant de séparer l'effluent de ladite section réactionnelle en au moins un effluent hydrogène sous forme gazeuse et un effluent éthanol/acétaldéhyde sous forme liquide.

Ladite section réactionnelle permet de convertir l'éthanol en acétaldéhyde en présence d'un catalyseur consistant de préférence en un mélange d'oxyde de chrome et d'oxyde de cuivre, ou de tout autre catalyseur adapté. Ces catalyseurs sont bien connus de l'Homme du métier.

Ladite section réactionnelle est opérée à une pression comprise entre 0,1 MPa et 1,0 MPa, de préférence entre 0,1 MPa et 0,5 MPa, de manière préférée entre 0,1 MPa et 0,3 MPa, et à une température comprise entre 200°C et 500°C, préférentiellement comprise entre 250°C et 300°C.

De préférence, la conversion de l'éthanol est comprise entre 30 et 40%, avec une sélectivité comprise entre 85 et 100% vers l'acétaldéhyde, de manière préférée comprise entre 90 et 95% vers l'acétaldéhyde. L'effluent de ladite section réactionnelle comprend également des sous-produits tels que le crotonaldéhyde, butyraldehyde, le diethylacetal, l'éthyl acétate et l'acide acétique.

Ladite section de séparation met en œuvre des moyens de séparation gaz-liquide connus de l'Homme du métier. De manière préférée, on utilisera un séparateur gaz-liquide opéré à une pression comprise entre 0,1 MPa et 0,3 MPa, et une température comprise entre 25°C et 60°C.

### Étape b2) de conversion d'un mélange éthanol/acétaldéhyde en butadiène

Conformément à un mode de réalisation selon l'invention, une étape b2) de conversion en butadiène comprend au moins une section réactionnelle alimentée au moins par une fraction dudit effluent éthanol/acétaldéhyde issu de l'étape b1), par un effluent liquide riche en éthanol issu de l'étape b3), par une fraction de l'effluent riche en acétaldéhyde issu de l'étape b5) et optionnellement avantageusement alimentée par un flux riche en éthanol issu de l'étape b5), et une section de séparation permettant de séparer l'effluent de ladite section réactionnelle en au moins un effluent gazeux et un effluent liquide. Ladite section réactionnelle peut également être alimentée par un flux externe d'acétaldéhyde.

Le débit des différentes alimentations de la section réactionnelle de ladite étape b2) est ajusté de telle sorte que le rapport molaire éthanol sur acétaldéhyde en entrée de ladite section réactionnelle est compris entre 1 et 5, de manière préférée compris entre 1 et 3,5, de manière encore plus préférée entre 2 et 3 et de manière très préférée entre 2,4 et 2,7.

Ladite section réactionnelle permet de convertir une partie du mélange éthanol/acétaldéhyde en au moins du butadiène. La sélectivité de la transformation du mélange éthanol/acétaldéhyde est de préférence supérieure à 60%, de manière préférée supérieure à 70%, de manière très préférée supérieure à 80%. Par sélectivité, on entend le rapport molaire du débit de butadiène dans l'effluent de ladite section réactionnelle sur le débit d'éthanol et d'acétaldéhyde consommé dans ladite section réactionnelle. La conversion de la transformation du mélange éthanol/acétaldéhyde est de préférence supérieure à 30%, de manière préférée supérieure à 40%, de manière préférée supérieure à 47%. Par conversion, on entend le rapport molaire du débit d'éthanol et d'acétaldéhyde dans l'effluent de ladite section réactionnelle sur le débit d'éthanol et d'acétaldéhyde dans l'alimentation de ladite section réactionnelle. Elle est opérée en présence d'un catalyseur, avantageusement un catalyseur supporté sur silice choisi dans le groupe constitué par les catalyseurs comprenant de l'oxyde de tantale, de zirconium ou de colombium, préférentiellement comprenant 2% d'oxyde de Tantale (voir par exemple Corson, Jones, Welling, Hincbley, Stahly, Ind. Eng Chem. 1950, 42, 2, 359-373). Ladite section réactionnelle est opérée à une température comprise entre 300°C et 400°C, de manière préférée entre 320°C et 370°C et à une pression comprise entre 0,1 MPa et 1,0 MPa, de préférence entre 0,1 MPa et 0,5 MPa, de manière préférée entre 0,1 MPa et 0,3 MPa.

De préférence, environ 65 à 80% de l'acétaldéhyde est converti dans ladite section réactionnelle. L'effluent de ladite section réactionnelle comprend donc encore de l'éthanol. De nombreuses impuretés peuvent être produites avec le butadiène, parmi lesquelles de l'éthylène, du propylène, du diéthyl ether (DEE), de l'acétate d'éthyle, du butanol, de l'hexanol, des butènes, des pentènes, des pentadiènes, des hexènes, et des hexadiènes.

Ladite section réactionnelle étant alimentée par l'effluent acétaldéhyde issu de l'étape b5) de traitement des effluents, le ratio éthanol sur acétaldéhyde à l'entrée de cette section est ajusté en contrôlant la fraction de l'effluent éthanol issu de ladite étape b5) alimentant l'étape b1), et donc produisant de l'acétaldéhyde. En effet, la fraction restante de l'effluent riche en éthanol issu de ladite étape b5) alimente l'étape b3) de traitement de l'hydrogène et forme, après lavage de l'effluent hydrogène, ledit effluent liquide riche en éthanol issu de ladite étape b3) qui alimente ladite étape b2). Or, cet effluent liquide riche en éthanol issu de ladite étape b3) ne contient que très peu d'acétaldéhyde.

Ladite section de séparation met en œuvre des moyens de séparation gaz-liquide connus de l'Homme du métier. De manière préférée, on utilisera un séparateur gaz-liquide opéré à une pression comprise entre 0,1 MPa et 0,3 MPa, et une température comprise entre 25°C et 60°C.

### Étape b3) de traitement de l'hydrogène

Conformément à un mode de réalisation selon l'invention, une étape b3) de traitement de l'hydrogène comprend au moins une section de compression alimentée par ledit effluent hydrogène issu de l'étape b1) et une section de lavage gaz-liquide alimentée par une fraction dudit effluent éthanol issu de ladite étape b5), et par une fraction dudit effluent éthanol/acétaldéhyde issu de ladite étape b1), et produit au moins un effluent liquide riche en éthanol et un effluent hydrogène purifié. De préférence, ladite étape b3) n'est alimentée par aucun autre flux.

Ladite fraction dudit effluent éthanol/acétaldéhyde issu de l'étape b1) est comprise entre 0 et 100%. L'utilisation d'une fraction dudit effluent éthanol/acétaldéhyde permet de diminuer le débit de la fraction dudit effluent riche en éthanol issu de ladite étape b5).

Ladite étape b3) permet d'obtenir un effluent hydrogène purifié très pur, c'est-à-dire comprenant au moins 90% molaire d'hydrogène, de préférence 99% molaire d'hydrogène, de préférence 99,8% molaire d'hydrogène. L'effluent hydrogène purifié comprend également des traces d'eau et d'éthanol. Cette étape permet également de récupérer l'éthanol et l'acétaldéhyde compris dans l'effluent hydrogène issu de ladite étape b1), permettant ainsi leur recyclage et maximisant le rendement global du procédé.

L'utilisation d'une fraction de l'effluent riche en éthanol issu de l'étape b5) et d'une fraction de l'effluent éthanol/acétaldéhyde issu de l'étape b1) en lieu et place d'eau - comme cela était réalisé dans l'art antérieur - permet de diminuer le débit total d'eau circulant dans le procédé. Ainsi, le débit total d'eau alimentant les étapes b5) et b5') de traitement des effluents est diminué, diminuant de ce fait la taille des équipements et la consommation en utilités des étapes b5) et b5'). De plus, comme cela a été décrit précédemment, l'effluent liquide riche en éthanol issu de ladite étape b3) peut alimenter directement l'étape b2) de conversion en butadiène sans avoir à être traité par l'étape b5) de traitement des effluents.

L'effluent hydrogène issu de l'étape b1) est comprimé dans une section de compression à une pression comprise entre 0,1 MPa et 1,0 MPa, avantageusement entre 0,1 MPa et 0,7 MPa, et de manière préférée entre 0,4 MPa et 0,68 MPa. L'effet de cette compression est d'une part de diminuer le débit volumique de gaz, et d'autre part d'améliorer l'efficacité du lavage en aval.

L'effluent hydrogène comprimé est ensuite refroidi à une température comprise entre 25 et 60°C, préférentiellement entre 30°C et 40°C, puis alimente en fond la colonne de lavage de la section de lavage dans laquelle il est mis en contact avec ladite fraction de l'effluent éthanol issu de l'étape b5) et ladite fraction de l'effluent éthanol/acétaldéhyde issu de l'étape b1), lesdites fractions étant respectivement alimentées en tête et en un point intermédiaire de ladite colonne de lavage. Ces deux fractions sont refroidies chacune à une température comprise entre 15°C et -30°C, préférentiellement entre 0°C et -15°C avant d'être alimentées dans ladite colonne de lavage. Avantageusement, l'effluent riche en éthanol issu de l'étape b5) sera alimenté à une température inférieure à celle de ladite fraction de l'effluent éthanol/acétaldéhyde issu de l'étape b1), créant ainsi un gradient thermique entre la tête et le fond et limitant les pertes en solvant dans l'effluent hydrogène purifié. La colonne de lavage gaz-liquide de la section de lavage est munie de plateaux ou de garnissage vrac ou structurés.

### Étape optionnelle b3') de traitement final de l'effluent hydrogène

Une étape b3') de traitement final de l'hydrogène est avantageusement réalisée à l'issue de l'étape b3). Ladite étape b3') comprend au moins d'une section de lavage gaz-liquide, alimentée par l'effluent hydrogène purifié issu de b3), et par un effluent eau pure d'origine externe au procédé ou par un effluent riche en eau issu de l'étape b5), et produit un effluent hydrogène purifié et un effluent eau usé.

Ladite section de lavage comprend au moins une colonne de lavage gaz-liquide, alimentée en fond par ledit effluent hydrogène purifié issu de b3), et en tête par un effluent eau pure ou par un effluent riche en eau issu de l'étape b5) et produisant un effluent hydrogène purifié en tête et un effluent eau usé en fond.

Cette étape permet de récupérer les dernières traces d'éthanol éventuellement contenues dans l'effluent hydrogène purifié issu de b3). Ladite étape b3'), similaire au traitement de l'hydrogène selon l'art antérieur, met néanmoins en œuvre des débits d'eau bien inférieurs à ceux utilisés dans l'art antérieur, l'effluent hydrogène alimentant l'étape b3') ayant été traité au préalable par l'étape b3), et donc débarrassé de la totalité de l'acétaldéhyde entrainé dans l'effluent hydrogène issu de l'étape b1). Toutes choses égales par ailleurs, l'élimination de traces d'éthanol par un lavage à l'eau demande des débits moindres que l'élimination de traces d'acétaldéhyde. De plus, l'éthanol étant moins volatil que l'acétaldéhyde, il est, dans les mêmes conditions opératoires, beaucoup moins entrainé que l'acétaldéhyde.

### Étape b4) d'extraction du butadiène

Conformément à un mode de réalisation selon l'invention, une étape b4) d'extraction du butadiène comprenant au moins une section de compression, une section de lavage gaz-liquide, et une section de distillation est alimentée au moins par lesdits effluents gazeux et liquide issus de ladite étape b2), par un flux éthanol constitué de ladite charge éthanol du procédé et/ou d'une fraction de l'effluent éthanol issu de l'étape b5), et produit au moins un effluent sous-produits gazeux, un effluent butadiène brut, et un effluent éthanol/acétaldéhyde/eau.

Ledit flux éthanol alimentant l'étape b4) comprend de préférence au moins 80% poids d'éthanol, préférentiellement au moins 90% poids, et de manière préférée au moins 93 % poids. Ledit flux éthanol alimentant l'étape b4) peut contenir du méthanol, de l'eau, de l'acétate d'éthyle, du butanol et de l'hexanol. De préférence, ledit flux éthanol alimentant l'étape b4) comprend moins de 10% poids d'acétaldéhyde, de préférence moins de 5% poids, et de manière préférée moins de 1% poids. De préférence, ledit flux éthanol alimentant l'étape b4) comprend moins de 20% poids d'eau, de préférence moins de 5% poids, de manière préférée moins de 1 % poids.

Dans un arrangement préféré, ledit flux éthanol alimentant l'étape b4) est constitué de ladite charge éthanol du procédé. Un avantage de cet arrangement est que ladite charge est exempte des sous-produits des réactions qui sont formés dans les étapes b1) et b2) et qui peuvent se trouver concentrés par le biais des recyclages. En particulier, cette charge éthanol ne contient pas d'acétaldéhyde, ou seulement à l'état de traces.

Dans un autre arrangement préféré, ledit flux éthanol est constitué d'une fraction de l'effluent éthanol issu de l'étape b5) de traitement des effluents.

L'utilisation d'un flux éthanol contenant peu ou pas d'acétaldéhyde minimise l'entraînement d'acétaldéhyde dans ledit effluent sous-produits gazeux soutiré en tête de ladite section de lavage gaz-liquide réduisant les pertes en rendement global du procédé, ainsi que le débit d'eau de lavage nécessaire dans l'étape b6).

L'effluent gazeux issu de l'étape b2) est comprimé dans ladite section de compression à une pression comprise entre 0,1 MPa et 1,0 MPa, préférentiellement entre 0,1 MPa et 0,7 MPa, et de manière préférée entre 0,2 MPa et 0,5 MPa. L'effet de cette compression est d'une part de diminuer le débit volumique de gaz, et d'autre part d'améliorer l'efficacité du lavage en aval. De préférence, l'effluent gazeux comprimé est ensuite refroidi à une température comprise entre 25°C et 60°C, préférentiellement entre 30°C et 40°C.

De préférence, ladite section de lavage gaz-liquide de l'étape b4) comprend une colonne de lavage alimentée en tête par ledit flux éthanol alimentant l'étape b4), en fond par ledit effluent gazeux comprimé et refroidi et produit en tête l'effluent sous-produits gazeux et en fond un effluent liquide qui alimente ladite section de distillation de l'étape b4).

Ledit flux éthanol alimentant l'étape b4) est refroidi avant d'être alimenté en tête de ladite colonne de lavage gaz-liquide de la section de lavage à une température comprise entre 20°C et -20°C, préférentiellement entre 15°C et 5°C. L'intérêt de refroidir ledit flux éthanol est d'améliorer la performance de l'opération de lavage en minimisant l'entraînement d'éthanol et d'acétaldéhyde dans ledit effluent sous-produits gazeux. Ainsi, la totalité du butadiène présent dans l'effluent gazeux issu de l'étape b2) comprimé et refroidi est abattue, et l'effluent sous-produits vapeur soutiré en tête de ladite section de lavage gaz-liquide est exempt de butadiène.

La minimisation de l'entraînement d'acétaldéhyde dans ledit effluent sous-produits gazeux permet, incidemment, de diminuer de manière importante le débit d'eau requis dans l'étape b6) de lavage à l'eau des sous-produits gazeux, dont l'objectif est de récupérer l'éthanol et les traces éventuelles d'acétaldéhyde entraînés dans l'effluent sous-produits gazeux soutiré en tête de la section de lavage à l'éthanol de l'étape b4).

De préférence, le flux éthanol enrichi en butadiène soutiré en fond de ladite section de lavage gaz-liquide de l'étape b4) ainsi que l'effluent liquide issu de l'étape b2) alimentent ladite section de distillation de l'étape b4) de manière à séparer en tête un effluent vapeur comprenant la majorité du butadiène, appelé effluent butadiène brut, et en fond un résidu éthanol/acétaldéhyde/eau. Par la majorité, on entend plus de 80% du butadiène compris dans l'alimentation de ladite section de distillation, préférentiellement plus de 90%, de manière préférée plus de 95%, de manière encore plus préférée plus de 98%, de manière très préférée plus de 99% et de manière très avantageuse la totalité du butadiène compris dans ladite alimentation. Ce résidu éthanol/acétaldéhyde/eau comprend de l'éthanol et de l'acétaldéhyde, et comprend également de l'eau produite dans l'étape b2) et des sous-produits formés dans les étapes b1) et b2), comme par exemple le diéthyléther et l'acétate d'éthyle et les huiles brunes. Ledit résidu éthanol/acétaldéhyde/eau alimente ensuite l'étape b5') de traitement des effluents. Ladite section de distillation est opérée à une pression comprise entre 0,1 MPa et 1 MPa et de manière préférée entre 0,2 MPa et 0,5 MPa.

### Etape b4') de première purification du butadiène

L'étape b4') de première purification du butadiène comprend au moins une section de lavage gaz-liquide alimentée en fond par l'effluent butadiène brut issu de b4) et en tête par un flux d'eau pouvant être un flux d'eau d'origine externe audit procédé de production de butadiène et/ou une fraction de l'effluent eau issu de l'étape b5), ladite section de lavage produisant en tête un effluent butadiène pré-purifié et en fond un effluent eau usée. De manière préférée, ledit flux eau est un flux d'eau d'origine externe au procédé.

Ledit effluent eau usée contient de l'acétaldéhyde et un peu de butadiène, et peut être envoyé vers l'étape b5) de traitement des effluents, vers la section de distillation de l'acétaldéhyde, ou vers l'étape b5').

L'objectif de l'étape b4') est d'éliminer les impuretés polaires, en particulier l'acétaldéhyde qui ne doit pas être présent au-delà de quelques ppm dans le butadiène final. L'effluent butadiène brut issu de b4) comprend la majorité du butadiène, mais contient encore de nombreuses impuretés, dont une quantité importante d'acétaldéhyde qui forme un azéotrope avec le butadiène et ne peut donc pas être complètement éliminé par distillation lors de l'étape b4). Ainsi, le débit dudit flux d'eau est ajusté pour obtenir la spécification recherchée en acétaldéhyde dans l'effluent butadiène pré-purifié.

Ledit flux d'eau est refroidi à une température inférieure à 25°C, de préférence inférieure à 20°C avant d'alimenter la section de lavage gaz-liquide de manière à faire le lavage avec une quantité d'eau réduite. La température d'alimentation dudit flux d'eau est choisie de manière à ne pas former d'hydrates avec le butadiène et les hydrocarbures légers encore présents dans le flux butadiène brut issu de l'étape b4). La pression de la colonne de lavage est déterminée de façon à assurer qu'il n'y ait aucune condensation du butadiène et qu'il reste bien sous forme gaz. La pression sur cette étape est comprise entre 0,1 MPa et 1 MPa, et de manière préférée entre 0,2 MPa et 0,3 MPa.

### Étape b4") ultérieure de purification du butadiène

Conformément à un mode de réalisation selon l'invention, une étape b4") ultérieure de purification du butadiène est alimentée au moins par ledit effluent butadiène pré-purifié issu de ladite étape b4'), et produit au moins un effluent butadiène purifié.

Cette étape b4") permet de purifier le butadiène produit dans les étapes réactionnelles à un très haut niveau de pureté (plus de 99,5% poids, préférentiellement plus de 99,8% poids, et très préférentiellement plus de 99,9% poids), tout en limitant les pertes de produit en séparant les impuretés n'ayant pas ou partiellement été retirées au cours de l'étape b4), b4').

Dans un premier mode de l'invention, ladite étape b4") comprend au moins une section de séchage, une section de distillation cryogénique et une section de séparation butadiène/butènes par extraction liquide-liquide.

L'effluent butadiène pré-purifié issu de l'étape b4') alimente une section de séchage. Cette section a pour objectif d'atteindre les spécifications requises en eau dans le produit final (effluent butadiène purifié), et de permettre de pratiquer une séparation cryogénique sans risque de formation d'hydrates. En sortie de ladite section de séchage, on obtient un effluent butadiène sec. Par butadiène sec on entend moins de 10 ppm d'eau, de préférence moins de 5 ppm, de préférence moins de 1 ppm.

Ladite section de séchage comprend préférentiellement un séchage constitué d'une ou plusieurs capacités contenant un ou plusieurs adsorbants ayant une forte affinité pour l'eau. De manière non limitative, cet adsorbant peut être constitué de silice et/ou d'alumine. De manière non limitative, cet adsorbant peut être une zéolite telle qu'une zéolite 3A ou 4A. Lorsque l'adsorbant ou les adsorbants sont saturés en eau, on alimente ledit effluent butadiène pré-purifié vers une autre capacité contenant l'adsorbant ou les adsorbants frais ou régénérés.

La régénération de l'adsorbant peut être réalisée soit en modifiant la pression partielle d'eau au sein de la capacité, soit en modifiant la température au sein de la capacité, soit en modifiant la pression partielle d'eau et la température au sein de la capacité. Dans ce dernier mode de réalisation, la régénération du ou des adsorbants saturés en eau est faite en chauffant la capacité, tout en l'alimentant avec un flux ne contenant pas, ou très peu, d'eau. Par pas ou très peu d'eau, on entend moins de 500 ppm, préférentiellement moins de 350 ppm, de manière préférée moins de 10 ppm, de préférence moins de 5 ppm, de manière très préférée moins de 1 ppm. Ce flux ne contenant pas ou très peu d'eau peut être de façon non limitative un flux d'azote, un flux d'air, un flux d'hydrocarbure, ou un flux d'hydrogène. Dans un mode préféré de l'invention, on utilise une fraction de l'effluent hydrogène purifié issu de l'étape b3).

Ledit flux ne contentant pas ou très peu d'eau est chauffée à une température suffisante pour régénérer l'adsorbant ou les adsorbants avant d'être alimentée dans la capacité contenant l'adsorbant ou les adsorbants à régénérer, préférentiellement aux environs de 250°C.

Selon ce premier mode de réalisation, ledit effluent butadiène sec alimente ensuite une section de distillation cryogénique mettant en œuvre une colonne à distiller. Les produits légers sortent en tête de la section de distillation cryogénique entre -25°C et -35°C. Le fond de la colonne est à une température comprise entre 20°C et 50°C, préférentiellement entre 25°C et 45°C, de manière très préférée entre 30°C et 40°C, la pression en tête de colonne est comprise entre 0,3 MPa et 0,4 MPa, préférentiellement 0,35 MPa. L'intérêt de la colonne est de présenter une très grande efficacité de séparation des derniers incondensables et ceci sans perte de butadiène (moins de 0,05 %). On évite ainsi un recyclage important vers l'étape b4) et une perte de butadiène.

Toujours selon ce premier mode de réalisation, le produit de fond de ladite section de distillation cryogénique, appelé effluent butadiène étêté, comprend pour principale impureté des butènes. Ledit effluent butadiène étêté alimente une section de séparation butadiène/butènes par extraction liquide-liquide, tel que décrite dans le brevet FR 2,036,057.

Ladite section de séparation butadiène/butènes est une section d'extraction liquide-liquide dans laquelle ledit effluent butadiène étêté alimente en une zone intermédiaire une première colonne d'extraction liquide-liquide, dans laquelle un flux de solvant polaire, préférentiellement du DMSO, est alimenté en tête. En fond, un solvant hydrocarbure saturé, préférentiellement du pentane ou du cyclohexane, est alimenté. Les débits ainsi que le ratio des débits de solvant polaire sur solvant hydrocarbure sont réglés de tel sorte que l'essentiel des butènes vont être entraînés par le solvant hydrocarbure et l'essentiel du butadiène être entraîné par le solvant polaire.

Le mélange butènes/hydrocarbure obtenu en tête de la première colonne d'extraction est ensuite traité dans une première colonne à distiller afin d'obtenir en tête l'effluent butènes, et en fond le solvant hydrocarbure qui peut être recyclé.

Le mélange butadiène/solvant polaire alimente ensuite la tête d'une deuxième colonne d'extraction liquide/liquide dans laquelle le butadiène est extrait du solvant polaire par mise en contact directe avec une quantité de solvant hydrocarbure plus importante que dans la première colonne d'extraction liquide-liquide, qui est introduite en fond de ladite deuxième colonne d'extraction liquide-liquide.

Le mélange butadiène/hydrocarbure obtenu en tête de la deuxième colonne d'extraction liquide-liquide est ensuite traité dans une colonne à distiller afin d'obtenir en tête l'effluent butadiène purifié, et en fond le solvant hydrocarbure qui peut être recyclé.

De manière préférée, les colonnes d'extraction liquide-liquide de ladite section de séparation butadiène/butènes sont opérées à une pression comprise entre 0,1 MPa et 1 MPa, et une température comprise entre 20°C et 60°C.

Dans un autre mode de l'invention, ladite étape b4") comprend au moins une distillation et une distillation extractive. L'étape de distillation peut être réalisée en amont ou en aval de l'étape de distillation extractive. La distillation extractive peut être réalisée de manière non limitative avec un solvant comme la N-methyl-pyrrolidone, le dimethyl-formamide ou l'acétonitrile.

### Étape b5) de traitement des effluents

Conformément à un mode de réalisation selon l'invention, l'étape b5) de traitement des effluents est alimentée au moins par le raffinat eau / éthanol / acétaldéhyde issu de l'étape b5') et produit au moins un effluent riche en éthanol, un effluent riche en acétaldéhyde et un effluent riche en eau. Si l'effluent eau usée issu de l'étape b4'), ou l'effluent eau alcoolisée issu de l'étape b6) ou l'effluent eau usée issu de l'étape b3') n'ont pas subi l'étape b5') d'élimination des impuretés et des huiles brunes, ils peuvent alimenter directement l'étape b5) de traitement des effluents. La section b5) est avantageusement également alimentée par une fraction de la charge éthanol.

De préférence et à la différence de l'art antérieur, aucun soutirage avec perte d'éthanol ou acétaldéhyde n'est effectué.

De préférence, ladite étape b5) comprend au moins deux sections de distillation. Une section de distillation dite de l'eau et de l'éthanol, et une section de distillation dite de l'acétaldéhyde.

Ledit effluent eau/éthanol/acétaldéhyde issu de l'étape b5') et éventuellement l'effluent eau usée issu de l'étape b4') alimentent ladite section de distillation de l'acétaldéhyde, dans laquelle l'acétaldéhyde est séparé de manière à former un effluent riche en acétaldéhyde, le résidu de ladite section de distillation de l'acétaldéhyde alimentant une section de distillation de l'eau et de l'éthanol permettant de séparer en tête un effluent riche en éthanol et en fond un effluent riche en eau. L'effluent eau alcoolisée issu de l'étape b6) et l'effluent eau usée issu de l'étape b3') ne contenant pas d'acétaldéhyde, ils peuvent alimenter directement ladite section de distillation de l'eau et de l'éthanol.

L'effluent riche en éthanol issu de l'étape b5) est constitué majoritairement d'éthanol. Par majoritairement, on entend plus de 80%pds, de préférence plus de 84 % poids. De manière non limitative, l'effluent riche en éthanol issu de l'étape b5) peut contenir des impuretés comme de l'eau, de l'acétate d'éthyle, du butanol et de l'hexanol.

Les impuretés autre que l'eau représentent moins de 10%, de façon privilégiée moins de 5%, de façon encore préférentielle moins de 2% poids du flux.

L'effluent riche en acétaldéhyde issu de l'étape b5) est constitué majoritairement d'acétaldéhyde et d'éthanol. Par majoritairement, on entend plus de 80% pds, de préférence plus de 85 % poids. De manière non limitative, l'effluent riche en acétaldéhyde issu de l'étape b5) peut contenir des impuretés comme de l'eau, de l'acétate d'éthyle, de l'acétone.

Les impuretés autre que l'eau représentent moins de 10%, de façon préférentielle moins de 5% poids du flux.

Lesdits effluents riches en acétaldéhyde, riche en éthanol et riche en eau sont ensuite recyclés dans le reste du procédé selon l'invention. La fraction dudit effluent riche en éthanol alimentant l'étape b1) est de préférence au moins de 0,7, de préférence au moins 0,75, de manière très préférée au moins 0,8. La fraction dudit effluent riche en eau alimentant ladite étape b6) est avantageusement compris entre 0 et 0,3, très avantageusement entre 0 et 0,1, plus avantageusement compris entre 0 et 0,01. La fraction dudit effluent riche en eau alimentant ladite étape b5') d'élimination des impuretés et des huiles brunes est avantageusement comprise entre 0 et 1, préférentiellement entre 0,3 et 0,6, et avantageusement entre 0,4 et 0,5.

Dans un autre mode de réalisation de l'invention, lesdits effluents riches en acétaldéhyde, riche en éthanol et riche en eau subissent une étape de purification avant d'être recyclés dans le reste du procédé. Par purification, on entend mettre en contact lesdits effluents avec des adsorbants comme par exemple du charbon actif, de la silice, de l'alumine ou encore une résine polymérique fonctionnalisée. Par exemple, un charbon actif permet d'éliminer les traces de butanol et d'hexanol comprises dans le flux riche en éthanol. Par exemple, une résine basique permet d'éliminer l'acide acétique présent dans l'effluent riche en eau. Quand les adsorbants sont saturés, et ne permettent pas de garantir la pureté des effluents riche en acétaldéhyde, riche en éthanol et riche en eau, ils sont soit éliminés, soit régénérés pour être réutilisés.

### Étape b5') d'élimination des impuretés liquides et des huiles brunes

Conformément à un mode de réalisation selon l'invention, une étape b5') d'élimination des impuretés et des huiles brunes est alimentée au moins par l'effluent éthanol/acétaldéhyde/eau issu de l'étape b4) et par une fraction de l'effluent eau issu de l'étape b5) et produit au moins un raffinat éthanol / acétaldéhyde / eau, un effluent huiles brunes légères et un effluent huiles brunes lourdes.

De préférence, ladite étape b5') comprend au moins une section de lavage/contre-lavage, une section de distillation des huiles brunes légères, et une section de distillation des huiles brunes lourdes.

Ladite section de lavage/contre-lavage préférentielle est alimentée en un point intermédiaire par ledit effluent éthanol/acétaldéhyde/eau issu de l'étape b4), avantageusement en mélange avec l'effluent eau usée issu de l'étape b4'), l'effluent eau alcoolisée issu de l'étape b6) et l'effluent eau usée issu de l'étape b3'), si cette dernière étape est mise en œuvre, et de manière préférée en mélange avec une fraction de l'effluent eau usée issu de l'étape b4'). Ces effluents étant plus riches en eau que l'effluent éthanol/acétaldéhyde/eau issu de l'étape b4), leur introduction en mélange permet de limiter les pertes en hydrocarbure dans le raffinat.

Ladite section de lavage/contre-lavage préférentielle est alimentée en fond par un effluent hydrocarbures et en tête par une fraction de l'effluent eau issue de l'étape b5), qui ne comprend pas d'éthanol et d'acétaldéhyde. L'effluent hydrocarbures et la fraction de l'effluent eau issu de l'étape b5) sont alimentés à une température de préférence comprise entre 10 et 70°C, préférentiellement entre 45°C et 55°C. Ladite section de lavage/contre-lavage produit en tête un extrait hydrocarbures de lavages chargée d'une fraction des impuretés et des huiles brunes, et en fond ledit raffinat éthanol / acétaldéhyde / eau.

Ladite section de lavage/contre-lavage est de préférence opérée à une pression comprise entre 0,1 MPa et 0,5 MPa, préférentiellement entre 0,2 MPa et 0,4 MPa. De préférence, l'ajout d'eau pour réaliser le contre-lavage est tel que la teneur en eau dans le raffinat eau / éthanol / acétaldéhyde est supérieure à 30% poids, de manière préférée supérieur à 40% poids.

Dans un mode de réalisation, le contact entre les deux phases liquides dans ladite section de lavage/contre-lavage est réalisé au sein d'un extracteur liquide-liquide. Différents modes de contact peuvent être envisagés. On peut citer de manière non limitative, une colonne garnie, une colonne pulsée, ou bien une colonne compartimentée agitée. Dans un autre mode de réalisation, le contact entre les deux phases liquides dans ladite section de lavage/contre-lavage est réalisé au sein d'un contacteur membranaire, ou une cascade de contacteurs membranaires. Ce mode de contact est particulièrement bien adapté au système mis en œuvre. **En** effet, les mélanges eau-éthanol-hydrocarbure sont connus pour former des émulsions stables, qui peuvent être problématique dans un extracteur liquide-liquide. Le contacteur membranaire permet de générer une aire de contact importante, favorisant le transfert des impuretés et des huiles vers la phase hydrocarbure, sans générer d'émulsion.

Ledit extrait hydrocarbures de lavages alimente ladite section de distillation des huiles brunes légères, laquelle produit en tant que distillat ledit effluent huiles brunes légères, et un résidu hydrocarbures comprenant la fraction lourde des huiles brunes.

Ledit effluent huiles brunes légères est composé d'impuretés produites par l'étape réactionnelle b2), principalement du diéthyléther, d'acétate d'éthyle et du crotonaldéhyde, mais aussi la fraction légère des huiles brunes, composée d'impuretés en quantité plus faible, parmi lesquelles du pentène, de l'isoprène, du butanal, du vinyl ethyl ether. Cet effluent peut être brulé pour fournir une partie de la chaleur nécessaire au circuit d'huile chaude ou aux chaudières à vapeur du procédé, ou distillé pour récupérer un effluent diéthyléther et/ou un effluent acétate d'éthyle/crotonaldéhyde, qui pourra être soit valorisée, soit recyclée dans la section réactionnelle de l'étape b2) pour être retransformé.

Ledit résidu hydrocarbures contient essentiellement les hydrocarbures servant au lavage, mais également la fraction la plus lourde des huiles brunes. Pour éviter l'accumulation des huiles brunes par le recyclage de l'effluent hydrocarbures vers l'extracteur liquide-liquide, une fraction dudit résidu hydrocarbures est traitée dans ladite section de distillation des huiles lourdes, consistant en une colonne à distiller, laquelle produit un distillat hydrocarbures composé pour l'essentiel d'hydrocarbures avec encore quelques traces d'huiles brunes et, en tant que résidu, ledit effluent huiles brunes lourdes comprenant plus de 80%, préférentiellement plus de 85% d'hydrocarbures ainsi que les huiles brunes les plus lourdes. La fraction dudit effluent hydrocarbures envoyée vers ladite section de distillation des huiles est comprise entre 5 et 30% du débit total dudit résidu hydrocarbures, et préférentiellement entre 10 et 20%. Le distillat hydrocarbures est mélangé à la fraction du résidu hydrocarbures qui n'a pas été traité dans ladite section de distillation des huiles lourdes afin de former l'effluent hydrocarbures alimentant ladite section de lavage/contre-lavage.

Cet effluent, qui représente de préférence entre 0,1 et 20% de la charge de ladite section de distillation des huiles lourdes, préférentiellement entre 0,3 et 5%, peut être brûlé pour fournir une partie de la chaleur nécessaire au circuit d'huile chaude ou aux chaudières à vapeur du procédé. Un appoint d'hydrocarbures équivalent aux pertes en fond de ladite section de distillation des huiles lourdes est nécessaire pour maintenir le débit de lavage constant. Cette colonne est réglée de manière à maintenir constante la concentration en huiles brunes dans la boucle de recyclage des hydrocarbures (boucle effluent hydrocarbures /effluent hydrocarbures de lavage).

Les effluents huiles brunes légères et lourdes sont éliminés du procédé.

L'effluent éthanol/acétaldéhyde/eau issu de l'étape b4) comprend principalement de l'éthanol, de l'acétaldéhyde, de l'eau mais aussi des impuretés telles que le diethyléther, l'acétate d'éthyle et les huiles brunes telles que définies précédemment. Ces impuretés peuvent s'accumuler si elles sont renvoyées vers les étapes réactionnelles b1) et b2) au sein de la coupe de distillation riche en acétaldéhyde et/ou de la coupe de distillation riche en l'éthanol et qu'elles ne sont que partiellement converties dans les sections réactionnelles des étapes b1) et b2). L'étape b5') permet de récupérer une partie de ces impuretés avant l'étape b5) de traitements des effluents, ce qui permet d'éviter la démixtion des huiles brunes au sein des colonnes à distiller, de simplifier le schéma de distillation, et d'obtenir à l'issue de l'étape b5) un effluent éthanol, un effluent acétaldéhyde et un effluent eau de plus grande pureté par rapport à l'art antérieur.

Le lavage de l'effluent éthanol/acétaldéhyde/eau issu de l'étape b4) avec un effluent hydrocarbures entraîne certaines impuretés, tandis que le contre-lavage du flux d'hydrocarbures entraine une partie des impuretés et des huiles brunes avec une fraction de l'effluent eau issu de l'étape b5), de manière à limiter toute perte en acétaldéhyde et en éthanol.

De manière surprenante, la demanderesse a découvert qu'il était possible d'obtenir une séparation de phase liquide-liquide en ajoutant certains hydrocarbures au résidu éthanol/acétaldéhyde issu de l'étape b4). Ce résultat est surprenant car le résidu éthanol/acétaldéhyde issu de l'étape est très riche en éthanol et acétaldéhyde qui sont miscibles en toute proportion avec les hydrocarbures. Par une sélection adéquate de l'hydrocarbure, la demanderesse a découvert qu'il était possible d'obtenir une séparation de phase liquide-liquide, et donc de réaliser une extraction liquide-liquide pour éliminer une partie des impuretés contenues dans l'effluent éthanol/acétaldéhyde/eau issu de l'étape b4). Ledit effluent hydrocarbures peut contenir des hydrocarbures saturés et/ou insaturés et /ou aromatiques, de préférence des hydrocarbures saturés. Ledit effluent hydrocarbures est avantageusement constitué d'un mélange d'hydrocarbures ayant entre 6 et 40 atomes de carbone, de préférence entre 10 et 20 atomes de carbone. De manière non limitative, ledit effluent hydrocarbures peut être une coupe gazole ou kérosène désulfurée ou bien encore une coupe d'hydrocarbures produite par une unité de type Fischer-Tropsh.

L'ajout d'eau au sein de la section de lavage/contre-lavage permet un meilleur fonctionnement du procédé d'élimination des impuretés et des huiles brunes selon l'invention.

### Étape b6) de lavage à l'eau des sous-produits gazeux

Conformément à un mode de réalisation selon l'invention, une étape b6) de lavage à l'eau est alimentée par l'effluent sous-produits gazeux issu de l'étape b4), ainsi que par une fraction de l'effluent riche en eau issu de ladite étape b5) et produit au moins un effluent eau alcoolisée.

L'objectif de ladite étape b6) est de récupérer la petite fraction d'éthanol entraînée dans ledit effluent sous-produits gazeux issu de l'étape b4) afin d'améliorer le rendement global du procédé.

La quantité d'eau issue de ladite étape b5) nécessaire dans ladite étape b6) selon l'invention est très faible, contrairement à celle nécessaire dans l'art antérieur, car l'effluent vapeur issu de l'étape b2) a été lavé dans l'étape b4) avec un flux éthanol contenant peu ou pas d'acétaldéhyde. Il ne reste donc dans ce flux qu'une petite fraction d'éthanol, facilement récupérée avec une faible quantité d'eau en comparaison avec la quantité d'eau qui aurait été nécessaire s'il y avait des traces d'acétaldéhyde dans l'effluent sous-produits gazeux issu de l'étape b4).

L'eau chargée en éthanol après le lavage est soutirée de ladite étape b6) et constitue l'effluent eau alcoolisée. Elle alimente de préférence l'étape b5), directement dans la section de distillation eau-éthanol sans alourdir la section de distillation de l'acétaldéhyde. Dans un autre mode de l'invention, elle alimente de préférence l'étape b5') d'élimination des impuretés et des huiles brunes.

L'étape b) du procédé selon l'invention permet de minimiser le débit des effluents à traiter dans l'étape de traitement des effluents et de réduire au maximum les pertes en butadiène, en permettant de récupérer plus de 98%, préférentiellement plus de 99 % du butadiène produit à l'issue des étapes réactionnelles dans ledit effluent butadiène purifié.

### Troisième section réactionnelle (Etape c) du procédé selon l'invention)

La troisième section réactionnelle **7** comprend au moins un réacteur dans lequel le 1,3-butadiène **6** issu de la deuxième section réactionnelle **5,** le CO₂ **4** issu de la première section réactionnelle **2** et l'hydrogène **9** de la section réactionnelle 5 sont introduits.

Selon un mode de réalisation préféré, l'étape c) du procédé selon l'invention comprend les sous-étapes suivantes :
c1) une étape de couplage C-C entre deux molécules de CO₂ et une molécule de 1,3-butadiène ;
c2) une étape de séparation des acides hexènedioïques ;
c3) une étape de réduction des diacides hexènedioïques formés dans à l'étape c1) en acide adipique.

### Etape c1)

Avantageusement, l'étape c1) est réalisée dans au moins un réacteur où les réactifs sont mis en contact avec un précurseur métallique, un ligand et un réducteur dans un solvant. Les réactifs sont le 1,3-butadiène issu de la deuxième section réactionnelle et le CO₂ produit dans la première section réactionnelle.

Le précurseur métallique est de préférence un sel de nickel (II). Plus préférentiellement, le sel de nickel est choisi parmi le sel de tetrabromonickelate de tetrabutylammonium (NiBr₄(DBA)₂), NiCl₂(DME), NiBr₂(DME).

Le ligand est de préférence un ligand bidentate diazoté (N,N) tels que des dérivées de bipyridine ou de phenanthroline. Plus préférentiellement, le ligand est choisi parmi le ligand 2-methyl-4,7-diphenyl-1,10-phenanthroline, 4,7-diphenyl-1,10-phenanthroline, 6-methyl-2,2'-bipyridine.

Le réducteur est de préférence un métal choisi parmi le zinc ou le manganèse. De préférence le réducteur est du manganèse. Il est aussi possible de réaliser l'étape de réduction avec un couple métal-photochimie ou électrochimie.

Le solvant est préférentiellement un solvant polaire aprotique. Le solvant a avantageusement une constante diélectrique à 20°C supérieure à 25,0 et/ou un moment dipolaire supérieur à 2,5 D. De manière préférée, le solvant est choisi parmi la N-méthylpyrrolidone (NMP), le N,N-diméthylformamide (DMF), le N,Ndiméthylacétamide (DMA), acétonitrile, diméthylsulfoxyde (DMSO), HMPT, hexaméthylphosphoramide (HMPA), nitrobenzène, formamide, nitrométhane, et le carbonate de propylène. De manière encore plus préférée, le solvant est le N,Ndiméthylacétamide (DMA).

L'étape c1) est réalisée avantageusement à une température comprise entre 5°C et 70°C, de préférence entre 40°C et 70°C.

Avant l'étape c2) séparation, le milieu réactionnel est de préférence neutralisé progressivement avec un acide, tel que le HCl, et avec de l'eau.

### Etape c2)

Durant l'étape c2) de séparation, les acides hexènedioïques sont avantageusement extraits du milieu aqueux avec de l'acétate d'éthyle via une extraction liquide-liquide. De préférence, l'effluent d'acétate d'éthyle enrichi en acides hexènedioïques est séché puis subit une distillation. Un des effluents est principalement de l'acétate d'éthyle qui peut être recyclé. Un autre effluent est enrichi en acides hexènedioïques qui peuvent être encore purifiés par cristallisation par exemple puis être dilués dans un solvant comme le méthanol pour alimenter la dernière étape.

### Etape c3)

L'étape c3) consiste à réduire les acides hexènedioïques en acide adipique. L'étape c3) peut être réalisée dans au moins un réacteur où les acides hexènedioïques dilués dans le méthanol sont introduits en présence d'un catalyseur et d'un agent de réduction.

Le catalyseur utilisé à l'étape c3) est de préférence un précurseur métallique sur un support ou un alliage de métaux, tel que le palladium sur charbon actif ou le nickel de Raney. Plus préférentiellement, le catalyseur est à base de palladium supporté sur charbon actif.

De manière préférentielle, l'hydrogène purifié provenant de l'étape b3) de la seconde section réactionnelle peut être utilisé à l'étape c3) en tant qu'agent de réduction. L'étape c3) de réduction est de préférence réalisée à une température comprise entre 10°C et 50°C, et plus préférentiellement entre 15°C et 25°C.

### Exemples

### 1 / Cas de la synthèse d'acide adipique à partir de glucose sans valorisation du CO2 de l'étape de fermentation

L'équation de la réaction de fermentation du glucose est C₆H₁₂O₆ → 2 C₂H₅OH + 2 CO₂. De ce fait, 66,7 % du carbone d'une molécule de glucose est converti en éthanol et 33,3 % en CO₂.

Pour une charge de 170 Kta de glucose, la fermentation permettrait théoriquement de produire 86,8 Kta d'éthanol et 83 Kta de CO₂.

En faisant l'hypothèse que le rendement massique de la transformation de l'éthanol en butadiène est de 40 %, il est possible de produire 34,7 Kta de butadiène, soit au mieux, 93,9 Kta d'acide adipique. Si le CO₂ n'est pas valorisé en acide adipique, jusqu'à 45,4% du carbone du glucose peut être valorisé en acide adipique (cf **Erreur ! Source du renvoi introuvable.**).

### 2/ Cas de la synthèse d'acide adipique à partir de glucose avec valorisation du CO2 de l'étape de fermentation

L'équation de la réaction de fermentation du glucose est C₆H₁₂O₆ → 2 C₂H₅OH + 2 CO₂. De ce fait, 66.7 % du carbone d'une molécule de glucose est converti en éthanol et 33,3 % en CO₂.

Pour une charge de 170 Kta de glucose, la fermentation permettrait théoriquement de produire 86,8 Kta d'éthanol et 83 Kta de CO₂.

En faisant l'hypothèse que le rendement massique de la transformation de l'éthanol en butadiène est de 40 %, il est possible de produire 34,7 Kta de butadiène, soit au mieux, 93,9 Kta d'acide adipique. Pour produire 93,9 Kta d'acide adipique, il faut au moins 56,6 Kta de CO₂. La fermentation produit donc assez de CO₂ pour que l'enchainement soit autosuffisant en CO₂. Si le CO₂ est valorisé en acide adipique, jusqu'à 68,1 % du carbone du glucose peut être valorisé en acide adipique (cf. **Erreur ! Source du renvoi introuvable.**).

**Table 1**

| Produits de valorisation de la biomasse | Production en Kt/a | % de C valorisable en acide adipique sans valorisation du CO₂ de la 1ère étape (non conforme) | % de C valorisable en acide adipique avec valorisation du CO₂ de la 1^{ère} étape (conforme) |
|---|---|---|---|
| Ethanol | 86,8 | 45,4% | 45,4% |
| CO₂ | 83,0 | | 22,7% |
| Total | | 45,4% | 68,1% |

## Revendications

1. Procédé de conversion de biomasse en acide adipique comprenant successivement :
a) une étape de traitement de la biomasse pour produire de l'éthanol et du CO₂ ;
b) une étape de conversion de l'éthanol obtenu à l'issue de l'étape a) pour obtenir du 1,3-butadiène et du dihydrogène ;
c) une étape de synthèse d'acide adipique à partir du 1,3-butadiène, du dihydrogène obtenus à l'issue de l'étape b) et du CO₂ obtenu à l'issue de l'étape a).

2. Procédé selon la revendication 1, dans lequel l'étape a) comprend les sous-étapes suivantes :
a1) une étape de prétraitement de la biomasse pour obtenir un substrat prétraité ;
a2) une étape d'hydrolyse enzymatique ou chimique du substrat prétraité obtenu à l'issue de l'étape a1) pour obtenir un moût d'hydrolyse enzymatique ou chimique ;
a3) une étape de fermentation alcoolique du moût d'hydrolyse enzymatique ou chimique obtenu à l'issue de l'étape a2) pour obtenir de l'éthanol et du CO₂.

3. Procédé selon la revendication 2, dans lequel la sous-étape a1) est réalisée par explosion à la vapeur en condition acide à une température comprise entre 150°C et 250°C et pendant une durée comprise entre 5 minutes et 30 minutes.

4. Procédé selon l'une des revendications 2 ou 3, dans lequel la sous-étape a2) est réalisée par hydrolyse enzymatique en présence des cellulases de *Trichoderma reesei.*

5. Procédé selon l'une quelconque des revendications 1 à 4, dans lequel l'étape b) comprend les sous-étapes suivantes :
b1) une étape de conversion de l'éthanol en acétaldéhyde comprenant au moins une section réactionnelle alimentée au moins par une fraction de l'effluent riche en éthanol issu de l'étape b5), opérée à une pression comprise entre 0,1 MPa et 1,0 MPa et à une température comprise entre 200°C et 500°C en présence d'un catalyseur, et une section de séparation permettant de séparer l'effluent de ladite section réactionnelle en au moins un effluent hydrogène sous forme gazeuse et un effluent éthanol/acétaldéhyde sous forme liquide ;
b2) une étape de conversion en butadiène comprenant au moins une section réactionnelle alimentée au moins par une fraction dudit effluent éthanol/acétaldéhyde issu de l'étape b1), par un effluent liquide riche en éthanol issu de l'étape b3), par une fraction de l'effluent riche en acétaldéhyde issu de l'étape b5), opérée en présence d'un catalyseur, à une température comprise entre 300°C et 400°C, et à une pression comprise entre 0,1 MPa et 1,0 MPa, les débits d'alimentation étant réglés de telle sorte que le rapport molaire éthanol/acétaldéhyde en entrée de ladite section réactionnelle est compris entre 1 et 5, et une section de séparation permettant de séparer l'effluent de ladite section réactionnelle en au moins un effluent gazeux et un effluent liquide ;
b3) une étape de traitement de l'hydrogène comprenant au moins une section de compression comprimant ledit effluent hydrogène issu de l'étape b1) à une pression comprise entre 0,1 MPa et 1,0 MPa, et une section de lavage gaz-liquide alimentée à une température comprise entre 15°C et -30°C par une fraction dudit effluent éthanol issu de l'étape b5) et par une fraction dudit effluent éthanol/acétaldéhyde issu de l'étape b1), et alimentée à une température comprise entre 25°C et 60°C par ledit effluent hydrogène comprimé, et produisant au moins un effluent liquide riche en éthanol et un effluent hydrogène purifié ;
b4) une étape d'extraction du butadiène comprenant au moins une section de compression comprimant ledit effluent gazeux issu de l'étape b2) à une pression comprise entre 0,1 MPa et 1,0 MPa, une section de lavage gaz-liquide comprenant une colonne de lavage alimentée en tête à une température comprise entre 20°C et -20°C par un flux éthanol constitué de ladite charge éthanol du procédé et/ou d'une fraction de l'effluent éthanol issu de l'étape b5) et en fond par ledit effluent gazeux issue de l'étape b2) et refroidi, et une section de distillation opérée à une pression comprise entre 0,1 MPa et 1 MPa, alimentée au moins par l'effluent liquide issu de ladite étape b2) et par l'effluent liquide de ladite section de lavage gaz-liquide, ladite étape b4) produisant au moins un effluent sous-produits gazeux, un effluent butadiène brut, et un effluent éthanol / acétaldéhyde / eau;
b4') une étape de première purification du butadiène comprenant au moins une section de lavage gaz-liquide alimentée en fond par l'effluent butadiène brut issu de b4) et en tête par un flux d'eau pouvant être un flux d'eau d'origine externe audit procédé de production de butadiène et/ou une fraction de l'effluent eau issu de l'étape b5), ladite section de lavage produisant en tête un effluent butadiène pré-purifié et en fond un effluent eau usée ;
b4") une étape ultérieure de purification du butadiène, alimentée au moins par ledit effluent butadiène pré-purifié issu de ladite étape b4'), et produisant au moins un effluent butadiène purifié ;
b5) une étape traitement des effluents alimentée au moins par le raffinat eau / éthanol / acétaldéhyde issu de l'étape b5'), et produisant au moins un effluent riche en éthanol, un effluent riche en acétaldéhyde et un effluent riche en eau ;
b5') une étape d'élimination des impuretés et des huiles brunes, alimentée au moins par l'effluent éthanol/acétaldéhyde/eau issu de l'étape b4), et par l'effluent riche en eau issu de l'étape b5), et produisant au moins un raffinat eau / éthanol / acétaldéhyde, un effluent huiles brunes légères et un effluent huiles brunes lourdes ;
b6) une étape de lavage à l'eau, alimentée par l'effluent sous-produits gazeux issu de l'étape b4), ainsi que par une fraction de l'effluent riche en eau issu de ladite étape b5) et produisant au moins un effluent eau alcoolisée.

6. Procédé selon l'une quelconque des revendications 1 à 5, dans lequel l'étape c) comprend les sous-étapes suivantes :
c1) une étape de couplage C-C entre deux molécules de CO₂ et une molécule de 1,3-butadiène ;
c2) une étape de séparation des acides hexènedioïques ;
c3) une étape de réduction des diacides hexènedioïques formés dans à l'étape c1) en acide adipique.

7. Procédé selon la revendication 6, dans lequel la sous-étape c1) est réalisée en présence d'un précurseur métallique, un ligand et un réducteur dans un solvant, **caractérisé en ce que** :
- le précurseur métallique est un sel de nickel (II);
- le ligand est un ligand bidentate diazoté ;
- le réducteur est un métal choisi parmi le zinc ou le manganèse ;
- le solvant est un solvant polaire aprotique.

8. Procédé selon l'une des revendications 6 ou 7, dans lequel la sous-étape c1) est réalisée à une température comprise entre 5°C et 70°C.

9. Procédé selon la revendication 6, dans lequel la sous-étape c3) est réalisée en présence d'un catalyseur et d'un agent de réduction, ledit catalyseur étant le palladium sur charbon actif, et l'agent de réduction étant le l'hydrogène purifié provenant de la sous-étape b3).

10. Procédé selon l'une quelconque des revendications précédentes, **caractérisé en ce que** la biomasse est une biomasse lignocellulosique.

11. Installation apte à mettre le procédé selon l'une quelconque des revendications 1 à 10, ladite installation comprenant :
- une première section réactionnelle (2) permettant de produire de l'éthanol (3) et du CO₂ (4) à partir de la biomasse (1) ;
- une deuxième section réactionnelle (5) de conversion de l'éthanol (3) en 1,3-butadiène (6) et en dihydrogène (9) ; et
- une troisième section réactionnelle (7) permettant de produire l'acide adipique (8) à partir du 1,3-butadiène (6), du CO₂ (4) et de dihydrogène (9).

## Patentansprüche

1. Verfahren zur Umsetzung von Biomasse zu Adipinsäure, das nacheinander Folgendes umfasst:
a) einen Schritt zur Behandlung der Biomasse zum Produzieren von Ethanol und CO₂;
b) einen Schritt zur Umsetzung des aus Schritt a) erhaltenen Ethanols, um 1,3-Butadien und molekularen Wasserstoff zu erhalten;
c) einen Schritt zur Synthese von Adipinsäure aus 1,3-Butadien und dem molekularen Wasserstoff, die aus Schritt b) erhalten werden, sowie aus dem aus Schritt a) erhaltenen CO₂.

2. Verfahren nach Anspruch 1, wobei Schritt a) die folgenden Teilschritte umfasst:
a1) einen Schritt zur Vorbehandlung der Biomasse, um ein vorbehandeltes Substrat zu erhalten;
a2) einen Schritt zur enzymatischen oder chemischen Hydrolyse des aus Schritt a1) erhaltenen vorbehandelten Substrats, um ein Hydrolysat aus einer enzymatischen oder chemischen Hydrolyse zu erhalten;
a3) einen Schritt zur alkoholischen Gärung des aus Schritt a2) erhaltenen Hydrolysats aus einer enzymatischen oder chemischen Hydrolyse, um Ethanol und CO₂ zu erhalten.

3. Verfahren nach Anspruch 2, wobei der Teilschritt a1) über eine Dampfexplosion unter sauren Bedingungen bei einer Temperatur zwischen 150 °C und 250 °C und während einer Dauer zwischen 5 Minuten und 30 Minuten durchgeführt wird.

4. Verfahren nach einem der Ansprüche 2 oder 3, wobei der Teilschritt a2) über eine enzymatische Hydrolyse in Gegenwart von Cellulasen von Trichoderma reesei durchgeführt wird.

5. Verfahren nach einem der Ansprüche 1 bis 4, wobei Schritt b) die folgenden Teilschritte umfasst:
b1) einen Schritt zur Umsetzung von Ethanol zu Acetaldehyd, der mindestens einen Reaktionsabschnitt, dem zumindest ein Anteil des Austrags mit hohem Ethanolanteil aus Schritt b5) zugeführt wird und der bei einem Druck zwischen 0,1 MPa und 1,0 MPa und bei einer Temperatur zwischen 200 °C und 500 °C in Gegenwart eines Katalysators betrieben wird, sowie einen Trennabschnitt umfasst, mit dem sich der Austrag aus dem Reaktionsabschnitt in mindestens einen Wasserstoffaustrag in gasförmiger Form und einen Ethanol/Acetaldehyd-Austrag in flüssiger Form auftrennen lässt;
b2) einen Schritt zur Umsetzung zu Butadien, der mindestens einen Reaktionsabschnitt umfasst, dem zumindest ein Anteil des Ethanol/Acetaldehyd-Austrags aus Schritt b1), ein flüssiger Austrag mit hohem Ethanolanteil aus Schritt b3), ein Anteil des Austrags mit hohem Acetaldehydanteil aus Schritt b5) zugeführt wird und der in Gegenwart eines Katalysators bei einer Temperatur zwischen 300 °C und 400 °C und bei einem Druck zwischen 0,1 MPa und 1,0 MPa durchgeführt wird, wobei der Volumenstrom beim Eintrag derart geregelt wird, dass das Molverhältnis Ethanol/Acetaldehyd am Eintritt des Reaktionsabschnitts zwischen 1 und 5 liegt, und einen Trennabschnitt umfasst, mit dem sich der Austrag aus dem Reaktionsabschnitt in mindestens einen gasförmigen Austrag und einen flüssigen Austrag auftrennen lässt;
b3) einen Schritt zur Behandlung von Wasserstoff, der mindestens einen Kompressionsabschnitt, in dem der Wasserstoffaustrag aus Schritt b1) bei einem Druck zwischen 0,1 MPa und 1,0 MPa komprimiert wird, und einen Gas-Flüssigkeit-Waschabschnitt umfasst, dem bei einer Temperatur zwischen 15 °C und -30 °C ein Anteil des Ethanolaustrags aus Schritt b5) und ein Anteil des Ethanol-/Acetaldehyd-Austrags aus Schritt b1) zugeführt wird, und dem bei einer Temperatur zwischen 25 °C und 60 °C der komprimierte Wasserstoffaustrag zugeführt wird und in dem mindestens ein flüssiger Austrag mit einem hohen Ethanolanteil und ein gereinigter Wasserstoffaustrag produziert wird;
b4) einen Schritt zur Extraktion von Butadien, der mindestens einen Kompressionsabschnitt, in dem der gasförmige Austrag aus Schritt b2) bei einem Druck zwischen 0,1 MPa und 1,0 MPa komprimiert wird, einen Gas-Flüssigkeit-Waschabschnitt, der eine Waschkolonne umfasst, der am Kopf bei einer Temperatur zwischen 20 °C und -20 °C ein Ethanolstrom zugeführt wird, der aus dem Ethanol-Einsatzmaterial des Verfahrens und/oder einem Anteil des Ethanolaustrags aus Schritt b5) besteht, und am Boden der abgekühlte gasförmige Austrag aus Schritt b2) zugeführt wird, und einen Destillationsabschnitt umfasst, der bei einem Druck zwischen 0,1 MPa und 1 MPa betrieben wird und dem zumindest der flüssige Austrag aus Schritt b2) und der flüssige Austrag aus dem Gas-Flüssigkeit-Waschabschnitt zugeführt wird, wobei in Schritt b4) mindestens ein Austrag mit gasförmigen Nebenprodukten, ein unbehandelter Butadienaustrag und ein Ethanol/Acetaldehyd/Wasser-Austrag produziert wird;
b4') einen Schritt zur ersten Reinigung von Butadien, der mindestens einen Gas-Flüssigkeit-Waschabschnitt umfasst, dem am Boden der unbehandelte Butadienaustrag aus b4) und am Kopf ein Wasserstrom zugeführt wird, der ein Wasserstrom sein kann, der von außerhalb des Verfahrens zur Produktion von Butadien stammt, und/oder ein Anteil des Wasseraustrags aus Schritt b5), wobei in dem Waschabschnitt am Kopf ein vorgereinigter Butadienaustrag und am Boden ein Abwasseraustrag produziert wird;
b4") einen weiteren Schritt zur Reinigung von Butadien, dem zumindest der vorgereinigte Butadienaustrag aus Schritt b4') zugeführt wird und in dem mindestens ein gereinigter Butadienaustrag produziert wird;
b5) einen Schritt zur Austragsbehandlung, dem zumindest das Wasser/Ethanol/Acetaldehyd-Raffinat aus Schritt b5') zugeführt wird und in dem mindestens ein Austrag mit einem hohen Ethanolanteil, ein Austrag mit einem hohen Acetaldehydanteil und ein Austrag mit einem hohen Wasseranteil produziert wird;
b5') einen Schritt zum Beseitigen von Verunreinigungen und braunen Ölen, dem zumindest der Ethanol/Acetaldehyd/Wasser-Austrag aus Schritt b4) und der Austrag mit hohem Wasseranteil aus Schritt b5) zugeführt wird und in dem mindestens ein Wasser/Ethanol/Acetaldehyd-Raffinat, ein Austrag mit leichten braunen Ölen und ein Austrag mit schweren braunen Ölen produziert wird;
b6) einen Schritt zum Waschen mit Wasser, dem der Austrag mit gasförmigen Nebenprodukten aus Schritt b4) sowie ein Anteil des Austrags mit hohem Wasseranteil aus Schritt b5) zugeführt wird und in dem mindestens ein Wasser-Alkohol-Austrag produziert wird.

6. Verfahren nach einem der Ansprüche 1 bis 5, wobei Schritt c) die folgenden Teilschritte umfasst:
c1) einen Schritt zur C-C-Kopplung zwischen zwei CO₂-Molekülen und einem 1,3-Butadien-Molekül;
c2) einen Schritt zur Abtrennung von Hexendisäuren;
c3) einen Schritt zur Reduktion der in Schritt c1) gebildeten Hexendisäuren zu Adipinsäure.

7. Verfahren nach Anspruch 6, wobei der Teilschritt c1) in Gegenwart einer metallischen Vorstufe, eines Liganden und eines Reduktionsmittels in einem Lösungsmittel durchgeführt wird, **dadurch gekennzeichnet, dass**:
- die metallische Vorstufe ein Nickel(II)-Salz ist;
- der Ligand ein zweizähniger diazotierter Ligand ist;
- das Reduktionsmittel ein Metall ist, das aus Zink oder Mangan ausgewählt ist;
- das Lösungsmittel ein aprotisches polares Lösungsmittel ist.

8. Verfahren nach Anspruch 6 oder 7, wobei der Teilschritt c1) bei einer Temperatur zwischen 5 °C und 70 °C durchgeführt wird.

9. Verfahren nach Anspruch 6, wobei der Teilschritt c3) in Gegenwart eines Katalysators und eines Reduktionsmittels durchgeführt wird, wobei der Katalysator Palladium auf Aktivkohle ist und das Reduktionsmittel der aus dem Teilschritt b3) stammende gereinigte Wasserstoff ist.

10. Verfahren nach einem der vorhergehenden Ansprüche, **dadurch gekennzeichnet, dass** die Biomasse eine lignocellulosehaltige Biomasse ist.

11. Anlage, die in der Lage ist, das Verfahren nach einem der Ansprüche 1 bis 10 auszuführen, wobei die Anlage Folgendes umfasst:
- einen ersten Reaktionsabschnitt (2), mit dem sich Ethanol (3) und CO₂ (4) aus Biomasse (1) produzieren lassen;
- einen zweiten Reaktionsabschnitt (5) zum Umsetzen von Ethanol (3) zu 1,3-Butadien (6) und zu molekularem Wasserstoff (9) und
- einen dritten Reaktionsabschnitt (7), mit dem sich Adipinsäure (8) aus 1,3-Butadien (6), CO₂ (4) und molekularem Wasserstoff (9) produzieren lässt.

## Claims

1. Process for converting biomass into adipic acid, successively comprising:
a) a step of treating the biomass to produce ethanol and CO₂;
b) a step of converting the ethanol obtained on conclusion of step a), to obtain 1,3-butadiene and dihydrogen;
c) a step of synthesizing adipic acid from the 1,3-butadiene and the dihydrogen obtained on conclusion of step b) and the CO₂ obtained on conclusion of step a).

2. Process according to Claim 1, wherein step a) comprises the following sub-steps:
a1) a step of pretreating the biomass to obtain a pretreated substrate;
a2) a step of enzymatic or chemical hydrolysis of the pretreated substrate obtained on conclusion of step a1) to obtain an enzymatic or chemical hydrolysis must;
a3) a step of alcoholic fermentation of the enzymatic or chemical hydrolysis must obtained on conclusion of step a2) to obtain ethanol and CO₂.

3. Process according to Claim 2, wherein substep a1) is carried out by steam explosion under acid conditions at a temperature of between 150°C and 250°C and for a period of between 5 minutes and 30 minutes.

4. Process according to either of Claims 2 and 3, wherein substep a2) is carried out by enzymatic hydrolysis in the presence of Trichoderma reesei cellulases.

5. Process according to any one of Claims 1 to 4, wherein step b) comprises the following substeps:
b1) a step of converting the ethanol into acetaldehyde, comprising at least a reaction section fed at least with a fraction of the ethanol-rich effluent resulting from step b5), operated at a pressure of between 0.1 MPa and 1.0 MPa and at a temperature of between 200°C and 500°C in the presence of a catalyst, and a separation section making it possible to separate the effluent of said reaction section into at least a gaseous hydrogen effluent and a liquid ethanol/acetaldehyde effluent;
b2) a step of converting into butadiene, comprising at least a reaction section fed at least with a fraction of said ethanol/acetaldehyde effluent resulting from step b1), with a liquid ethanol-rich effluent resulting from step b3), with a fraction of the acetaldehyde-rich effluent resulting from step b5), operated in the presence of a catalyst at a temperature of between 300°C and 400°C and at a pressure of between 0.1 MPa and 1.0 MPa, the feed flow rates being adjusted so that the ethanol/acetaldehyde molar ratio at the inlet of said reaction section is between 1 and 5, and a separation section making it possible to separate the effluent of said reaction section into at least a gaseous effluent and a liquid effluent;
b3) a step of treating the hydrogen, comprising at least a compression section compressing said hydrogen effluent resulting from step b1) to a pressure of between 0.1 MPa and 1.0 MPa and a gas/liquid scrubbing section fed at a temperature of between 15°C and -30°C with a fraction of said ethanol effluent resulting from step b5) and with a fraction of said ethanol/acetaldehyde effluent resulting from step b1) and fed at a temperature of between 25°C and 60°C with said compressed hydrogen effluent, and producing at least a liquid ethanol-rich effluent and a purified hydrogen effluent;
b4) a step of extracting the butadiene, comprising at least a compression section compressing said gaseous effluent resulting from step b2) to a pressure of between 0.1 MPa and 1.0 MPa, a gas/liquid scrubbing section comprising a scrubbing column fed at the top, at a temperature of between 20°C and -20°C, with an ethanol stream consisting of said ethanol feedstock from the process and/or a fraction of the ethanol effluent resulting from step b5) and at the bottom with said gaseous effluent resulting from step b2), which is cooled, and a distillation section operated at a pressure of between 0.1 MPa and 1 MPa, fed at least with the liquid effluent resulting from said step b2) and with the liquid effluent from said gas/liquid scrubbing section, said step b4) producing at least one gaseous by-products effluent, a crude butadiene effluent and an ethanol/acetaldehyde/water effluent;
b4') a step of first purification of the butadiene, comprising at least a gas/liquid scrubbing section fed at the bottom with the crude butadiene effluent resulting from b4) and at the top with a stream of water which may be a stream of water which originates outside said process for producing butadiene and/or a fraction of the water effluent resulting from step b5), said scrubbing section producing a pre-purified butadiene effluent at the top and a waste water effluent at the bottom;
b4'') a subsequent step of purifying the butadiene, which is fed at least with said pre-purified butadiene effluent resulting from said step b4') and producing at least a purified butadiene effluent;
b5) a step of treating the effluents, which is fed at least with the water/ethanol/acetaldehyde raffinate resulting from step b5'), and producing at least an ethanol-rich effluent, an acetaldehyde-rich effluent and a water-rich effluent;
b5') a step of removing impurities and brown oils, which is fed at least with the ethanol/acetaldehyde/water effluent resulting from step b4) and with the water-rich effluent resulting from step b5), and producing at least a water/ethanol/acetaldehyde raffinate, a light brown oils effluent and a heavy brown oils effluent;
b6) a step of washing with water, which is fed with the gaseous by-products effluent resulting from step b4) and also with a fraction of the water-rich effluent resulting from said step b5), producing at least one alcoholic water effluent.

6. Process according to any one of Claims 1 to 5, wherein step c) comprises the following substeps:
c1) a step of C-C coupling between two molecules of CO₂ and one molecule of 1,3-butadiene;
c2) a step of separating out hexenedioic acids;
c3) a step of reducing the hexenedioic diacids formed in step c1) to give adipic acid.

7. Process according to Claim 6, wherein substep c1) is carried out in the presence of a metal precursor, a ligand and a reducing agent in a solvent, **characterized in that**:
- the metal precursor is a nickel(II) salt;
- the ligand is a bidentate dinitrogen ligand;
- the reducing agent is a metal selected from zinc or manganese;
- the solvent is a polar aprotic solvent.

8. Process according to either of Claims 6 and 7, wherein substep c1) is carried out at a temperature of between 5°C and 70°C.

9. Process according to Claim 6, wherein substep c3) is carried out in the presence of a catalyst and a reducing agent, said catalyst being palladium on activated carbon, and the reducing agent being the purified hydrogen originating from substep b3).

10. Process according to any one of the preceding claims, **characterized in that** the biomass is a lignocellulosic biomass.

11. Plant suitable for implementing the process according to any one of Claims 1 to 10, said plant comprising:
- a first reaction section (2) making it possible to produce ethanol (3) and CO₂ (4) from biomass (1);
- a second reaction section (5) for converting the ethanol (3) into 1,3-butadiene (6) and dihydrogen (9); and
- a third reaction section (7) making it possible to produce adipic acid (8) from 1,3-butadiene (6), CO₂ (4) and dihydrogen (9).
